# EUROPEAN PATENT SPECIFICATION

(11) **EP 3 144 020 B1**
(45) Date of publication and mention of the grant of the patent: **21.08.2019**
(21) Application number: 15758837.7
(22) Date of filing: 24.04.2015
(51) Int. Cl.: A61M 1/16, A61M 1/34, F04B 9/02, F04B 23/06, G01F 11/02

(54) **BLOOD PURIFICATION DEVICE**
BLUTREINIGUNGSVORRICHTUNG
DISPOSITIF DE PURIFICATION DU SANG

(30) Priority: 15.05.2014 JP 2014101538; 27.05.2014 JP 2014109547; 27.05.2014 JP 2014109541; 25.09.2014 JP 2014195371
(43) Date of publication of application: 22.03.2017
(73) Proprietor: Nipro Corporation, Osaka-shi, Osaka 531-8510 (JP)
(72) Inventor: UEDA, Mitsutaka, Osaka-shi Osaka 531-8510 (JP); MATSUMOTO, Tomoyuki, Osaka-shi Osaka 531-8510 (JP); OKADA, Shogo, Osaka-shi Osaka 531-8510 (JP); NAKANO, Hiroomi, Tokyo 170-0001 (JP)
(74) Representative: Neusser, Sebastian
(86) International application number: PCT/JP2015/062588
(87) International publication number: WO 2015/133653

(56) References cited:
- WO-A2-2005/044339
- JP-A- H07 299 133
- JP-A- S62 155 859
- JP-A- 2001 234 850
- JP-A- 2001 234 850
- JP-A- 2007 222 548
- JP-B2- 3 322 987
- US-A- 5 158 441
- US-A1- 2005 013 708

## Description

### Technical Field

The present invention relates to a blood purification device in which a pair of plunger pumps are disposed in a dialysate line.

More specifically, the present invention relates to a blood purification device which enables supply of a fresh dialysate without pulsation to a blood purifier.

### Background Art

Heretofore, in order to improve serious states of patients with renal failure, patients developing hyperhydration due to chemical feeding after an operation, and the like, hemodialysis or blood filtration using a blood purification device has been performed. In order to supply a fresh dialysate to a blood purifier and discharge a used dialysate from a blood purifier, one utilizing a reciprocating pump capable of simultaneously performing the supply of a fresh dialysate and the discharge of a used dialysate is known heretofore (Patent Literature 1, Patent Literature 2).

Moreover, a plunger pump not requiring a valve for extracting the air in a dialysate has also been suggested as a reciprocating pump (Patent Literature 3, Patent Literature 4) .

### Citation List

### Patent Literatures

Patent Literature 1: Japanese Patent No. 3328078
Patent Literature 2: Japanese Patent Laid-Open No. 2003-199820
Patent Literature 3: Japanese Patent Laid-Open No. 2001-248543
Patent Literature 4: Japanese Patent Laid-Open No. 2001-234850

### Summary of Invention

### Technical Problem

The reciprocating pumps described in Patent Literatures 1 and 2 additionally require a water removing pump and a pressurization pump in order to perform water removal and backfiltration, and thus have posed a problem of an increase in cost. The reciprocating pumps described in Patent Literatures 3 and 4 do not simultaneously perform the supply of a fresh dialysate and the discharge of a used dialysate. Therefore, the reciprocating pumps need to be improved so as to simultaneously perform the supply of a fresh dialysate and the discharge of a used dialysate. Moreover, a plunger pump which enables the supply of a fresh dialysate without pulsation to a blood purification device has been desired.

JP 2001234850 discloses a valveless plunger pump comprising two plungers allowing to pump fluid without pulsation when the phase difference of the rotation angle of the plunger is 180°.

JP 3322987 B discloses a blood purification device with a blood purifier, a blood circuit, and a blood pump.

WO 2005/044339 A2 discloses a system for performing a renal replacement therapy in which two dialyzers are connected fluidly in series.

The present invention has been made in view of the above-described circumstances. It is an object of the present invention to provide a means simultaneously performing the supply of a fresh dialysate and the discharge of a used dialysate in a dialysate line.

### Solution to Problem

The problem is solved by the features of the independent claims.

A blood purification device according to the present invention has a blood purifier, a blood circuit connected to the blood purifier, a blood pump for generating blood flow in the blood circuit, a dialysate line having a fresh dialysate supply line and a used dialysate discharge line individually connected to the blood purifier, and plunger pumps individually provided in the fresh dialysate supply line and the used dialysate discharge line so as to form one pair. The plunger pump provided in the fresh dialysate supply line and the plunger pump provided in the used dialysate discharge line are synchronized so that the delivery of a fresh dialysate by the plunger pump provided in the fresh dialysate supply line and the suction of a used dialysate by the plunger pump provided in the used dialysate discharge line are simultaneously performed. A stroke of one of the plunger pumps of the pair of plunger pumps individually provided in the fresh dialysate supply line and the used dialysate discharge line is variable and can be made larger and smaller than the stroke of the other plunger pump.

The blood purification device further has a synchronization motor and drive joints which rotate by driving force given from the synchronization motor, in which the pair of plunger pumps may be disposed in a mirror-symmetrical manner with respect to the plane orthogonal to the rotation shaft of the synchronization motor and may be connected to the synchronization motor by the drive joints.

In the plunger pump in which the stroke is variable of the pair of plunger pumps, the inclination angle of a shaft of a plunger with respect to the rotation shaft of the synchronization motor may be adjustable.

An angle adjustment motor varying the inclination angle of the shaft of the plunger may be provided.

The stroke of the plunger pump provided in the used dialysate discharge line may be variable.

The rotation radius of the drive joint on the used dialysate suction side may be larger than the rotation radius of the drive joint on the fresh dialysate delivery side.

The plunger pump may be a valveless plunger pump.

Another pair of plunger pumps having a 180° shifted phase may be disposed in parallel to the dialysate line.

Another blood purification device according to the present invention has a blood purifier, a blood circuit connected to the blood purifier, a blood pump for generating blood flow in the blood circuit, a dialysate line having a fresh dialysate supply line and a used dialysate discharge line which are individually connected to the blood purifier so as to be at least partially in parallel to each other, and a pair of plunger pumps provided in each of the parallel fresh dialysate supply line and the parallel used dialysate discharge line. The pair of plunger pumps individually provided in the parallel fresh dialysate supply lines alternately and continuously perform the delivery of a fresh dialysate by one plunger pump of the pair of plunger pumps and the delivery of a fresh dialysate by the other plunger pump. The pair of plunger pumps provided in the parallel used dialysate discharge lines alternately and continuously perform the suction of a used dialysate by one plunger pump of the pair of plunger pumps and the suction of a used dialysate by the other plunger pump. The pair of plunger pumps provided in the fresh dialysate supply line and the pair of plunger pumps provided in the used dialysate discharge line are synchronized so that the delivery of a fresh dialysate by the pair of plunger pumps provided in the fresh dialysate supply line and the suction of a used dialysate by the pair of plunger pumps provided in the used dialysate discharge line are simultaneously performed. A stroke of one of the plunger pumps of the pair of plunger pumps provided in the parallel used dialysate discharge lines is variable and can be made larger and smaller than the strokes of the pair of plunger pumps provided in the fresh dialysate supply line.

In the pair of plunger pumps of the parallel fresh dialysate supply line, the delivery of a fresh dialysate from one plunger pump and the delivery of a fresh dialysate from the other plunger pump are alternately and continuously performed. In the pair of plunger pumps of the parallel used dialysate discharge line, the suction of a used dialysate into one plunger pump and the suction of a used dialysate to the other plunger pump are alternately and continuously performed. Further, the plunger pumps are synchronized so that the delivery of a fresh dialysate and the suction of a used dialysate simultaneously occur. Therefore, the supply of a fresh dialysate to the blood purifier and the discharge of a used dialysate from the blood purifier are simultaneously performed. Thus, a fresh dialysate without pulsation is supplied to the blood purifier.

The stroke of at least one of the pair of plunger pumps provided in the parallel used dialysate discharge lines is variable.

Thus, when the strokes are adjusted so that the strokes of the plunger pumps provided in the used dialysate discharge line are smaller than the strokes of the plunger pumps provided in the fresh dialysate supply line, backfiltration can be performed in the blood purifier. When the strokes are adjusted so that the strokes of the plunger pumps provided in the used dialysate discharge line are larger than the strokes of the plunger pumps provided in the fresh dialysate supply line, water removal can be performed in the blood purifier.

Preferably, a synchronization motor and a first drive joint which rotates by driving force given from the synchronization motor are further provided, and the pair of plunger pumps provided in the parallel fresh dialysate supply lines are disposed in a mirror-symmetrical manner with respect to the plane orthogonal to the rotation shaft of the synchronization motor and are connected to the synchronization motor by the first drive joint.

The pair of plunger pumps are disposed in a mirror-symmetrical manner with respect to the plane orthogonal to the rotation shaft of the synchronization motor and are individually connected to the rotation shaft of the synchronization motor via the first drive joint, and therefore the pair of plunger pumps have a relationship in which the phases are 180° shifted. Therefore, even when the phases of the pair of plunger pumps are not adjusted, the supply of a fresh dialysate to the blood purifier can be alternately and continuously performed.

Preferably, a synchronization motor and a second drive joint which rotates by driving force given from the synchronization motor are further provided, and the pair of plunger pumps provided in the parallel used dialysate discharge lines are disposed in a mirror-symmetrical manner with respect to the plane orthogonal to the rotation shaft of the synchronization motor and are connected to the synchronization motor by the second drive joint.

The pair of plunger pumps are disposed in a mirror-symmetrical manner with respect to the plane orthogonal to the rotation shaft of the synchronization motor and are individually connected to the rotation shaft of the synchronization motor via the second drive joint, and therefore the pair of plunger pumps have a relationship in which the phases are 180° shifted. Thus, even when the phases of the pair of plunger pumps are not adjusted, the discharge of a used dialysate from the blood purifier can be alternately and continuously performed.

Preferably, the pair of plunger pumps provided in the parallel fresh dialysate supply lines and the pair of plunger pumps provided in the parallel used dialysate discharge lines are disposed in a mirror-symmetrical manner with respect to the plane orthogonal to the rotation shaft of the synchronization motor.

Thus, the pair of plunger pumps have a relationship in which the phases are 180° shifted. Therefore, even when the phases of the pair of plunger pumps are not adjusted, the supply of a fresh dialysate to the blood purifier and the discharge of a used dialysate from the blood purifier can be simultaneously performed.

Preferably, a synchronization motor and a second drive joint which rotates by driving force given from the synchronization motor are further provided, and the pair of plunger pumps provided in the parallel used dialysate discharge lines are connected to the synchronization motor by the second drive joint and, in the plunger pump in which the stroke is variable of the pair of plunger pumps individually provided in the parallel used dialysate discharge lines, the inclination angle of a shaft of a plunger with respect to the rotation shaft of the synchronization motor is adjustable.

The stroke of the plunger pump provided in the used dialysate discharge line can be adjusted by adjusting the inclination angle between the plunger pump and the rotation shaft of the synchronization motor.

Preferably, an angle adjustment motor varying the inclination angle of the shaft of the plunger is further provided.

Thus, also when the plunger pump is driven, the inclination angle of the shaft of the plunger can be adjusted.

As described above, the present invention is generally described and can be further understood by referring to some specific examples. These examples are presented herein for purposes of illustration, and not limited unless otherwise specified.

### Advantageous Effects of Invention

According to the present invention, supply of a fresh dialysate to a blood purifier and discharge of a used dialysate can be simultaneously performed.

Moreover, a blood purification device in which a pair of plunger pumps capable of performing water removal and backfiltration are disposed is realized.

Moreover, supply of a fresh dialysate and discharge of a used dialysate can be simultaneously performed to a dialysate line and a fresh dialysate without pulsation can be supplied to a blood purifier.

### Brief Description of Drawings

Fig. 1 is a schematic explanatory view of a blood purification device 10 according to a first embodiment of the present invention.
Fig. 2 is a schematic plan view illustrating the configuration in the vicinity of plunger pumps 51 and 52.
Fig. 3 is a schematic side view illustrating the configuration in the vicinity of the plunger pump 51 and 52.
Fig. 4 is a diagram showing the relationship between the swing angle θ and the stroke d2 of the plunger pump 52 (Drive radius of 29.5 mm).
Fig. 5 is a schematic plan view illustrating a modification of the plunger pumps 51 and 52.
Fig. 6 is a diagram showing the relationship between the swing angle θ and the stroke d2 of the plunger pump 52 in a modification of the first embodiment (Drive radius of 34.5 mm).
Fig. 7 is a schematic view illustrating the structure of a blood purification device 10 according to a second embodiment of the present invention.
Fig. 8 is a plan view illustrating the structure of plunger pumps 51, 52, 53, and 54.
Fig. 9 is a side view illustrating the structure of the plunger pumps 51, 52, 53, and 54.
Fig. 10 is a schematic view illustrating a modification of the blood purification device 10.
Fig. 11 is a schematic view illustrating a modification of the blood purification device 10.

### Description of Embodiments

Hereinafter, in order to further specify the present invention, embodiments of the present invention are described with reference to the drawings.

### [First embodiment]

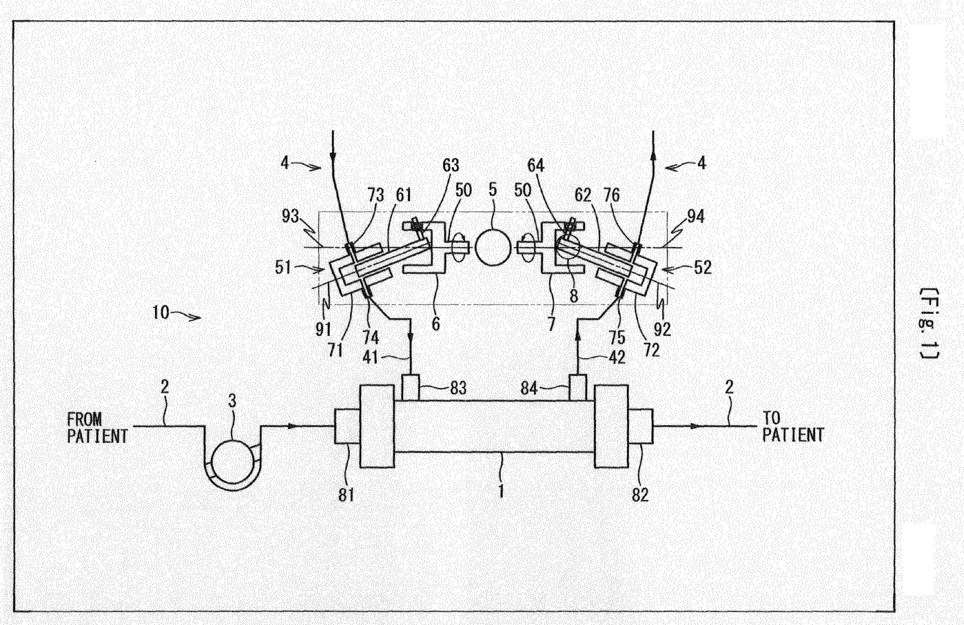

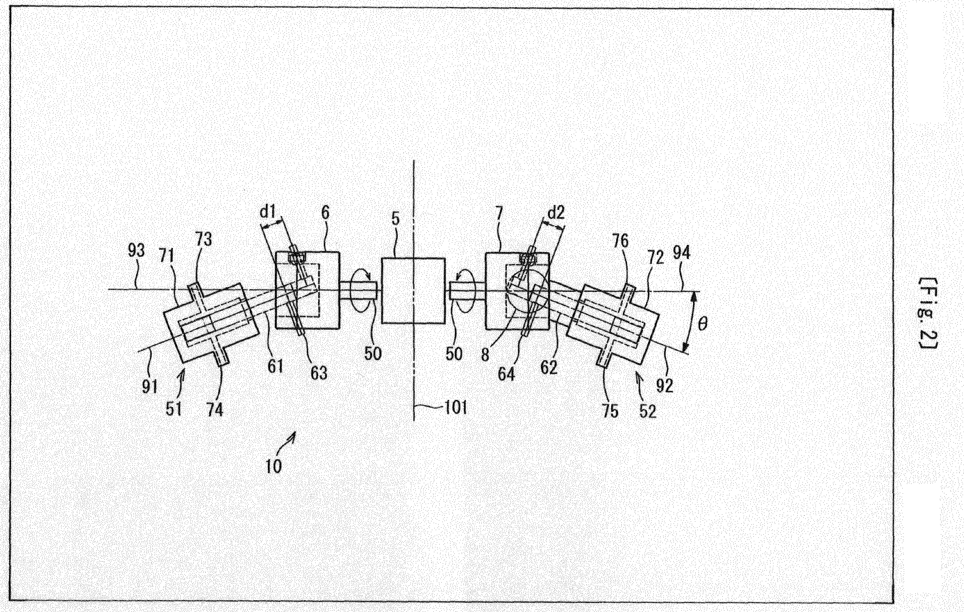

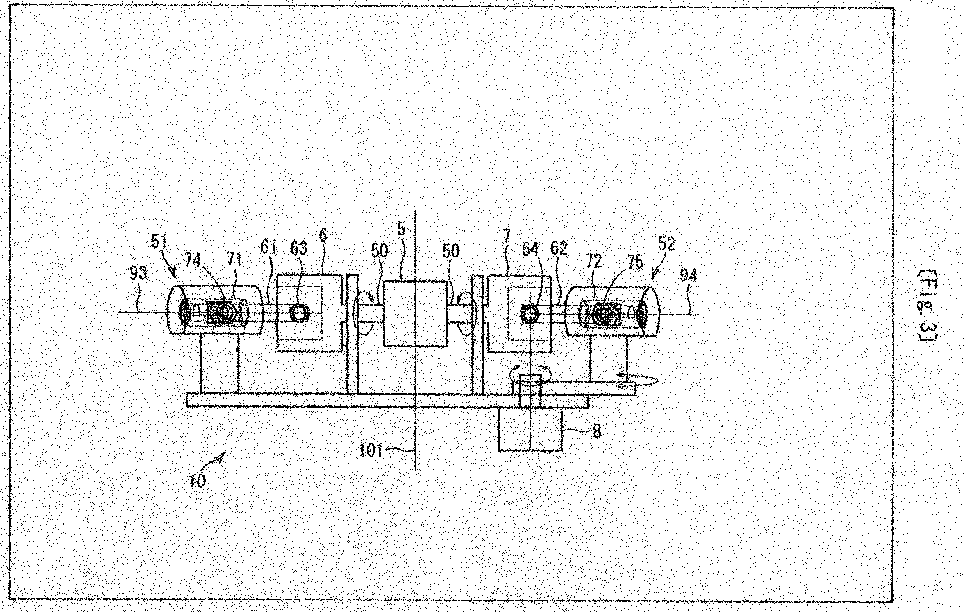

Fig. 1 illustrates a schematic explanatory view illustrating a first embodiment. Fig. 2 and Fig. 3 illustrates a schematic plan view and a schematic side view, respectively, illustrating one example of the plunger pump of Fig. 1.

A blood purification device 10 contains a blood purifier 1, a blood circuit 2, a blood pump 3, and a dialysate line 4 having a fresh dialysate supply line 41 and a used dialysate discharge line 42 as illustrated in Fig. 1. In the dialysate line 4, a pair of plunger pumps 51 and 52 are disposed.

The blood purifier 1 is a container which has a first port 81, a second port 82, a third port 83, and a fourth port 84 for inflow and outflow of blood and a dialysate and the inside of which is filled with hollow fibers. Due to the fact that blood is caused to pass through the internal space of the hollow fibers through the first port 81 and the second port 82 and a dialysate is caused to pass through the outside of the hollow fibers through the third port 83 and the fourth port 84, removal of water from blood or backfiltration is performed.

The blood circuit 2 is connected to the first port 81 and the second port 82. The blood circuit 2 forms a blood flow passage containing a resin tube or the like and leads blood flowing out of a blood vessel of a patient to the blood purifier 1 and leads blood flowing out of the blood purifier 1 to a blood vessel of a patient. The blood circuit 2 is provided with the blood pump 3 for generating blood flow in the blood circuit 2. For the blood pump 3, known substances, such as a tube pump, may be employed.

The dialysate line 4 is connected to the third port 83 and the fourth port 84 of the blood purifier 1. The dialysate line 4 forms a dialysate flow passage containing a resin tube or the like. The fresh dialysate supply line 41 is connected to the third port 83 of the blood purifier 1 and the used dialysate discharge line 42 is connected to the fourth port 84 of the blood purifier 1. Although not illustrated in each figure, the other end of the fresh dialysate supply line 41 is connected to a tank in which a fresh dialysate is stored and the other end of the used dialysate discharge line 42 is connected to a waste tank storing a used dialysate.

The plunger pump 51 is disposed in the fresh dialysate supply line 41. The plunger pump 52 is disposed in the used dialysate discharge line 42.

To the pair of plunger pumps 51 and 52, rotation is transmitted from a rotation shaft 50 of a synchronization motor 5 through drive joints 6 and 7. The drive joints 6 and 7 are connected to one end side of plungers 61 and 62 of the plunger pumps 51 and 52, respectively. The plungers 61 and 62 reciprocate in cylinders 71 and 72 by drive transmitted from the drive joints 6 and 7, respectively.

Axial directions 91 and 92 of the plungers 61 and 62 incline (cross) with respect to axial directions 93 and 94 of the drive joints 6 and 7, respectively. In this embodiment, the axial directions 93 and 94 of the drive joints 6 and 7 are the same as the axial direction of a rotation shaft 50. The strokes of the plungers 61 and 62 which reciprocate by drive transmitted from the drive joints 6 and 7, respectively, are determined depending on the inclination angle θ between the axial directions 91 and 92 of the plungers 61 and 62 and the axial directions 93 and 94 of the drive joints 6 and 7, respectively. More specifically, when the inclination angle θ is large, the strokes of the plungers 61 and 62 become large and, when the inclination angle θ is small, the strokes of the plungers 61 and 62 become small.

A pair of ports 73 and 74 or a pair of ports 75 and 76 communicating with the internal space are provided in cylinders 71 and 72, respectively. The pair of ports 73 and 74 and the pair of ports 75 and 76 are disposed at positions different by 180° with respect to the axial directions of the cylinders 71 and 72, respectively, i.e., axial symmetry. Although not illustrated in detail in each figure, the plungers 61 and 62 have a columnar shape sealing the cylinder 71 and 72, respectively, in a fluid-tight manner and the half including the axis line of the columnar shape on the tip side (other end side which is not connected to the drive joints 6 and 7) is notched. Due to the fact that the notched portions rotate in the cylinders 71 and 72, one of the pair of ports 73 and 74 or the pair of ports 75 and 76 of the cylinders 71 and 72 is sealed by the plungers 61 and 62, respectively, and the other port is opened by the notched portion.

As illustrated in Figs. 1 to 3, the one pair of plunger pumps 51 and 52 have a mirror-symmetrical structure with respect to the plane (plane orthogonal to the sheet of Figs. 1 to 3) orthogonal to the rotation shaft 50 of the synchronization motor 5. In detail, as illustrated in Figs. 2 and 3, the plunger pumps 51 and 52 have a mirror-symmetrical structure with respect to a plane 101 orthogonal to the rotation shaft 50 of the synchronization motor 5 and including the middle in a direction along the axial directions 93 and 94 of the drive joints 6 and 7, respectively. Therefore, the angles at which the plungers 61 and 62 incline with respect to the drive joints 6 and 7, respectively, when the angles θ are the same, the positions of the ports 73, 74, 75, and 76 of the cylinders 71 and 72, respectively, and the like are in a mirror-symmetrical state.

As the mirror-symmetrical structure, the structures of the plungers 61 and 62 and the cylinders 71 and 72 are in a mirror-symmetrical state. However, a state where, by making the angle θ variable, the axial directions 91 and 92 of the plungers 61 and 62 of the plunger pumps 51 and 52, respectively, are not in a mirror-symmetrical state and a state where, due to the fact that the rotation radii of the drive joints 6 and 7 are different from each other, a strict mirror-symmetrical state is not achieved are not excluded from the mirror-symmetrical structure. More specifically, the mirror-symmetrical structure is to be understood as follows: insofar as a relationship in which the phases of the pair of plunger pumps 51 and 52 are 180° shifted can be maintained when the pair of plunger pumps 51 and 52 are connected through the drive joints 6 and 7, respectively, so as to be rotated by the same synchronization motor 50, it is permitted that the angles, the rotation radii, and the like are different from each other in the pair of plunger pumps 51 and 52.

Thus, the phases of the plunger pumps 51 and 52 are different from each other by 180° with respect to the rotation of the drive joints 6 and 7. More specifically, the plunger pump 52 is sucking a fresh dialysate while the plunger pump 51 is delivering a fresh dialysate and the plunger pump 52 is delivering a fresh dialysate while the plunger pump 51 is sucking a fresh dialysate. Thus, the pair of plunger pumps 51 and 52 simultaneously perform the delivery of a fresh dialysate by the plunger pump 51 and the delivery of a fresh dialysate by the plunger pump 52 in a synchronized manner.

Due to the fact that the plungers 61 and 62 reciprocate in the cylinders 71 and 72 in the plunger pumps 51 and 52, respectively, a fresh dialysate is delivered in the plunger 51 and a used dialysate is sucked in the plunger 52. Since the uniform rotation of the synchronization motor 50 is transmitted as the stroke of each plunger 61 and 62 by the drive joints 6 and 7, respectively, the movement speed of each of the plungers 61 and 62 forms a sin curve or a cos curve with respect to the rotation phase of the drive joints 6 and 7, respectively. Therefore, the delivery amount of a fresh dialysate by the plunger 51 and the suction amount of a used dialysate by the plunger 52 form a sin curve or a cos curve. The fluctuation of the delivery amount of a fresh dialysate by the plunger 51 and the fluctuation of the suction amount of a used dialysate by the plunger 52 represented by such a sin curve or a cos curve are referred to as "pulsation" in this specification. Since the delivery of a fresh dialysate by the plunger pump 51 and the delivery of a fresh dialysate by the plunger pump 52 are simultaneously performed in a synchronized manner, the "pulsation" thereof simultaneously arise in a synchronized manner.

The pair of plunger pumps 51 and 52 are synchronized so that the delivery of a fresh dialysate from the plunger pump 51 (delivery step) and the suction of a used dialysate into the plunger pump 52 (suction step) simultaneously occur, and the stroke of at least one of the pair of plunger pumps 51 and 52 is made variable. Therefore, the supply of a fresh dialysate to the blood purifier 1 and the discharge of a used dialysate from the blood purifier 1 can be simultaneously performed. When a stroke d1 of the plunger pump 51 on the fresh dialysate delivery side is made larger than a stroke d2 of the plunger pump 52 on the used dialysate suction side, backfiltration can be performed. When the stroke d1 of the plunger pump 51 on the fresh dialysate delivery side is made smaller than the stroke d2 of the plunger pump 52 on the used dialysate suction side, water removal can be performed.

Specifically, as the pair of plunger pumps 51 and 52, the pair of plunger pumps 51 and 52 are disposed in a mirror-target manner with respect to the rotation shaft 50 of the synchronization motor 5 and are connected to the synchronization motor 5 located in the central portion of the rotation shaft 50 through the drive joints 6 and 7, respectively, as illustrated in Fig. 2 and Fig. 3, for example.

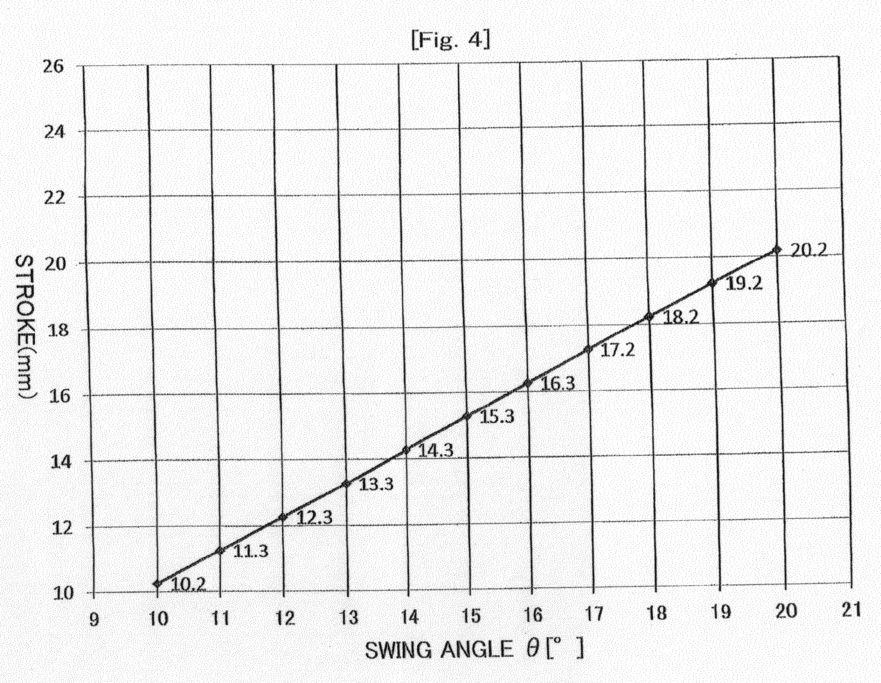

This embodiment is configured so that the stroke d1 of the plunger pump 51 is fixed, the stroke d2 of the plunger pump 52 is made variable, and the adjustment of the stroke d2 of the plunger pump 52 is performed by the angle adjustment motor 8 adjusting the horizontal inclination angle θ between the axial direction 91 of the plunger pump 52 and the rotation shaft 94 of the synchronization motor 5. Herein, the inclination angle θ can be adjusted so that the stroke d2 can be made larger or can be made smaller than the stroke d1 as illustrated in Fig. 4. When the inclination angle θ is made larger, the stroke becomes larger. When the inclination angle θ is made smaller, the stroke becomes smaller. In this connection, when the relationship between the stroke and the delivery amount of the plunger pump when the plunger diameter was 16 mm was determined, the delivery amount per 1 mm stroke was about 24.1 cc/min.

Herein, the adjustment function of the inclination angle of the plunger pump is described in a little more detail. More specifically, the drive joints 6 and 7 are provided with a bearing socket. To the bearing socket, a bearing is attached. The bearings each are provided with a through-hole in the central portion. One end of operation shafts 63 and 64 extending from the plungers 61 and 62 of the plunger pumps 51 and 52, respectively, is slidably inserted into and passed through the through-holes. This embodiment is configured so that the operation shafts 63 and 64 are fixed so that that the other end thereof is perpendicular to the surface of the plungers 61 and 62, respectively, and that the rotation of the synchronization motor 5 is transmitted to the drive joints 6 and 7, the rotation of the drive joints 6 and 7 is transmitted to the plungers 61 and 62 by the operation shafts 63 and 64, respectively, and the stroke d1 and d2 are generated in the inclined plunger pumps 51 and 52, respectively. Therefore, the plungers 61 and 62 are configured so as to reciprocate according to the stroke d1 and d2 while rotating in the cylinders 71 and 72, respectively.

The arrangement manner of the pair of plunger pumps 51 and 52 with respect to the rotation axes of the drive joints 6 and 7 and the rotation shaft 50 of the synchronization motor 5 is not limited. In any case, in order to simultaneously cause the delivery of a fresh dialysate from the plunger pump 51 and the suction of a used dialysate into the plunger pump, the phases of the plunger pumps 51 and 52 need to be 180° shifted.

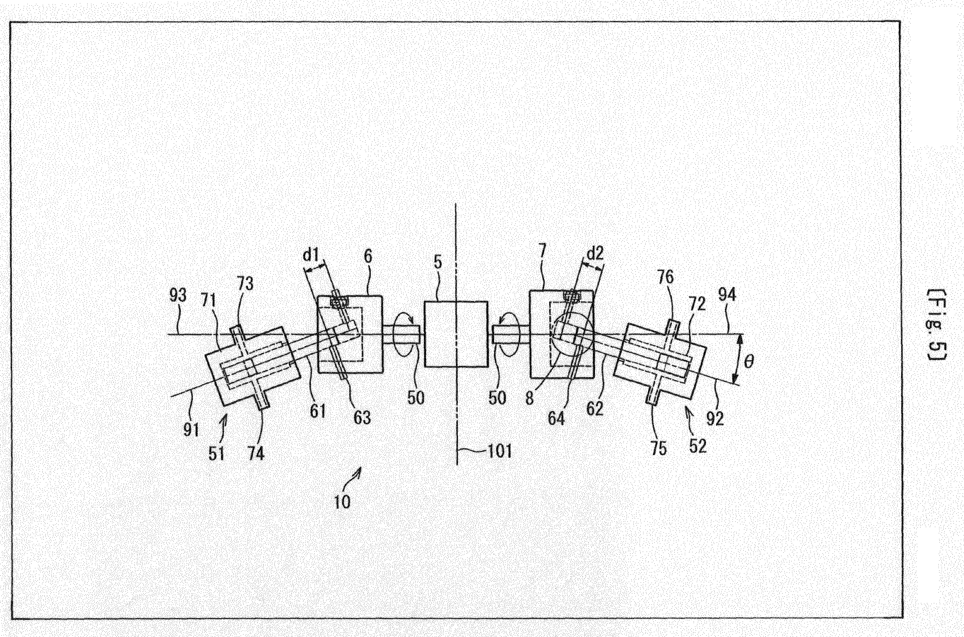

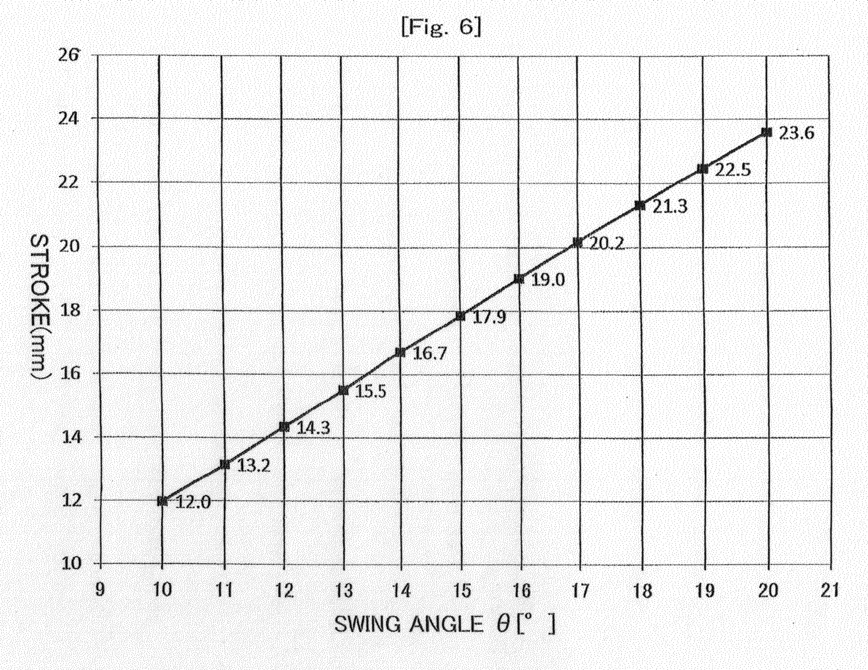

As the pair of plunger pumps 51 and 52 disposed in the dialysate line 4, the rotation radius of the drive joint 7 on the used dialysate suction side may be made larger than the rotation radius of the drive joint 6 on the fresh dialysate delivery side and the stroke d2 of the plunger pump 52 on the used dialysate suction side may be made variable as illustrated in Fig. 5. When configured as described above, the stroke d2 of the plunger pump 52 on the used dialysate suction side can be greatly varied as illustrated in Fig. 6. Therefore, when the suction amount corresponding to the delivery amount of the plunger pump 51 is set, the inclination angle θ of the plunger pump 52 can be made smaller than the inclination angle of the plunger pump 51, and thus the adjustment of the inclination angle θ of the plunger pump 52 is facilitated.

As the plunger pumps 51 and 52, a valveless plunger pump may be employed. In this case, a valve for extracting the air in a dialysate may not be separately prepared, and therefore the economical efficiency is achieved.

### [Operational effects of first embodiment]

According to the first embodiment, the pair of plunger pumps 51 and 52 disposed in the dialysate line 4 are synchronized so that the delivery of a fresh dialysate from the plunger pump 51 and the suction of a used dialysate into the plunger pump 52 simultaneously occur, and therefore the supply of a fresh dialysate to the blood purifier 1 and the discharge of a used dialysate from the blood purifier 1 can be simultaneously performed.

Moreover, the stroke of the plunger pump 52 is made variable, and therefore, when the stroke d1 of the plunger pump 51 is made larger than the stroke d2 of the plunger pump 52, backfiltration can be performed and, when the stroke d1 of the plunger pump 51 is made smaller than the stroke d2 of the plunger pump 52, water removal can be performed.

Moreover, the pair of plunger pumps 51 and 52 are disposed in a mirror-target manner on the rotation shaft 50 of the synchronization motor 5 and are connected through the drive joints 6 and 7 so as to be rotated by the same synchronization motor 5, and therefore, the pair of plunger pumps 51 and 52 have a relationship in which the shifts are 180° shifted from the beginning, and, even when the shifts thereof are not adjusted, the supply of a fresh dialysate to the blood purifier 1 and the discharge of a used dialysate from the blood purifier can be simultaneously performed.

Moreover, the stroke d1 of the plunger pump 51 is fixed and the horizontal angle θ of the plunger pump 52 is variable with respect to the rotation shaft 50 of the synchronization motor 5 by the angle adjustment motor 8, and therefore the stroke d2 of the plunger pump 52 can be adjusted by varying the horizontal angle θ of the plunger pump 52 to be made larger or to be made small than the stroke d1 of the plunger pump 51. Therefore, backfiltration or water removal can be performed by adjusting the stroke d2 of the plunger pump 52.

Moreover, the rotation radius of the drive joint 7 is made larger than the rotation radius of the drive joint 6, and therefore the stroke d2 of the plunger pump 52 can be greatly varied, so that the angle adjustment of the plunger pump 52 is facilitated (A smaller inclination angle is acceptable.). Moreover, as compared with a case where the drive joints 6 and 7 of both the plunger pumps 51 and 52 are made to have the same size, a large backfiltration amount and a large water removal amount can be set.

Moreover, a valveless plunger pump is employed as the plunger pumps 51 and 52, and therefore a valve for extracting the air in a dialysate can be omitted.

The plunger pumps 51 and 52 are preferably formed with glass . The plungers 61 and 62 and the cylinders 71 and 72 each are preferably manufactured using shrinking processing. Due to the fact that the plunger pumps 51 and 52 are manufactured from glass, the sealability in the plunger pumps 51 and 52 can be secured even when the tolerance of the inner diameter of the cylinders 71 and 72 to the outer diameter of the plungers 61 and 62 is large to some extent, and therefore the mass productivity of the plunger pumps 51 and 52 is improved. In each of the plunger pumps 51 and 52, both the plungers 61 and 62 and the cylinders 71 and 72 are preferably formed with glass . Materials of the plunger pumps 51 and 52 are not limited to glass and the plunger pumps 51 and 52 may be formed with other materials, such as ceramics. A method for manufacturing the plungers 61 and 62 and the cylinders 71 and 72 is not limited to the method employing shrinking processing.

The delivery step of delivering a fresh dialysate to the blood purifier 1 through the fresh dialysate supply line 41 by transmitting rotational drive to the plunger pump 51 from the synchronization motor 50 and the suction step of sucking a used dialysate from the blood purifier 1 through the used dialysate discharge line 42 by transmitting rotational drive to the plunger pump 52 from the synchronization motor 50 are simultaneously performed in a synchronized manner, and therefore the supply of a fresh dialysate and the discharge of a used dialysate to/from the blood purifier 1 can be simultaneously performed using the pair of plunger pumps 51 and 52.

Moreover, the transmission of the drive of the synchronization motor 50 to the plunger pump 51 and the transmission of the drive of the synchronization motor 50 to the plunger pump 52 are performed at a 180° shifted phase, and therefore the phase of the pulsation generated when supplying a fresh dialysate to the blood purifier 1 by the plunger pump 51 and the phase of the pulsation generated when discharging a used dialysate from the blood purifier 1 by the plunger pump 52 are synchronized, so that the pressure of the dialysate in the blood purifier 1 is stabilized.

Moreover, by differentiating the stroke d1 of the plunger pump 51 and the stroke d2 of the plunger pump 52 from each other, backfiltration of blood or removal of water from blood can be performed in the blood purifier 1.

### [Second embodiment]

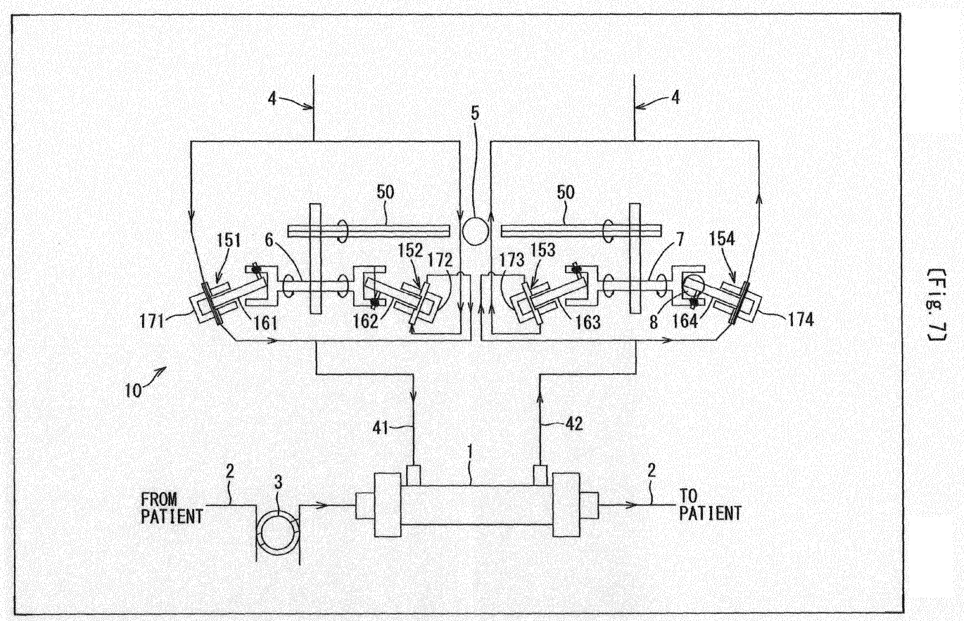

As illustrated in Fig. 7, a blood purification device 11 has a blood purifier 1, a blood circuit 2 connected to the blood purifier 1, a blood pump 3 generating blood flow in the blood circuit 2, a dialysate line 4 having a fresh dialysate supply line 41 and a used dialysate discharge line 42, plunger pumps 151, 152, 153, and 154, and a synchronization motor 5.

The blood purifier 1 is a container which has inflow and outflow ports of blood and a dialysate and the inside of which is filled with hollow fibers . Due to the fact that blood is caused to pass through the internal space of the hollow fibers through the blood inflow and outflow ports and a dialysate is caused to pass through the outside of the hollow fibers through the dialysate inflow and outflow ports, removal of water from blood or backfiltration is performed.

The blood circuit 2 is connected to the blood inflow and outflow ports of the blood purifier 1. The blood circuit 2 forms a blood flow passage containing a resin tube or the like and leads blood flowing out of a blood vessel of a patient to the blood purifier 1 and leads blood flowing out of the blood purifier 1 to a blood vessel of a patient. The blood circuit 2 is provided with the blood pump 3 for generating blood flow in the blood circuit 2. For the blood pump 3, known substances, such as a tube pump, may be employed.

The dialysate line 4 is connected to the dialysate inflow and outflow ports of the blood purifier 1. The dialysate line 4 forms a dialysate flow passage containing a resin tube or the like. The fresh dialysate supply line 41 is connected to the inflow port of the blood purifier 1 and the used dialysate discharge line 42 is connected to the outflow port of the blood purifier 1. Although not illustrated in each figure, the other end of the fresh dialysate supply line 41 is connected to a tank in which a fresh dialysate is stored and the other end of the used dialysate discharge line 42 is connected to a waste tank storing a used dialysate.

In the fresh dialysate supply line 41, a part of the line between the blood purifier 1 and a tank (not illustrated) has a parallel structure in which the line is divided into two lines. In the fresh dialysate supply line 41 forming the parallel structure, plunger pumps 151 and 152 are individually disposed in the parallel lines so as to form one pair.

To the pair of plunger pumps 151 and 152, rotation is transmitted through a drive joint 6 (an example of the first drive joint) from a rotation shaft 50 of the synchronization motor 5. The drive joint 6 is connected to one end side of plungers 161 and 162 of the plunger pumps 151 and 152, respectively. The plungers 161 and 162 reciprocate in cylinders 171 and 172, respectively, by drive transmitted from the drive joint 6. Axial directions 191 of the plungers 161 and 162 incline (cross) with respect to an axial direction 193 of the drive joint 6. The strokes of the plungers 161 and 162 which reciprocate by drive transmitted from the drive joint 6 are determined depending on the inclination angle θ between the axial directions 191 of the plungers 161 and 162 and the axial direction 193 of the drive joint 6. More specifically, when the inclination angle θ is large, the strokes of the plungers 161 and 162 become large and when the inclination angle θ is small, the strokes of the plungers 161 and 162 become small.

A pair of ports communicating with the internal space are provided in each of the cylinders 171 and 172. The pair of ports are disposed at positions different by 180° with respect to the axial directions of the cylinders 171 and 172, i.e., axial symmetry. Although not illustrated in detail in each figure, the plungers 161 and 162 have a columnar shape sealing the cylinder 171 and 172, respectively, in a fluid-tight manner and the half including the axis line of the columnar shape on the tip side (other end side which is not connected to the drive joint 6) is notched. Due to the fact that the notched portions rotate in the cylinders 171 and 172, one of the pair of ports of the cylinders 171 and 172 is sealed by the plungers 161 and 162, respectively, and the other port is opened by the notched portion.

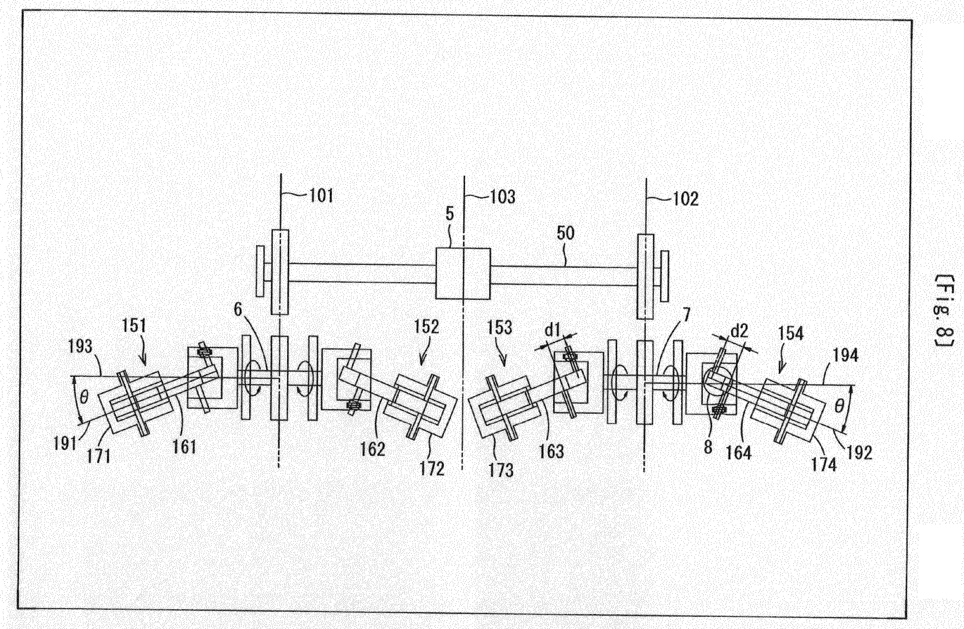

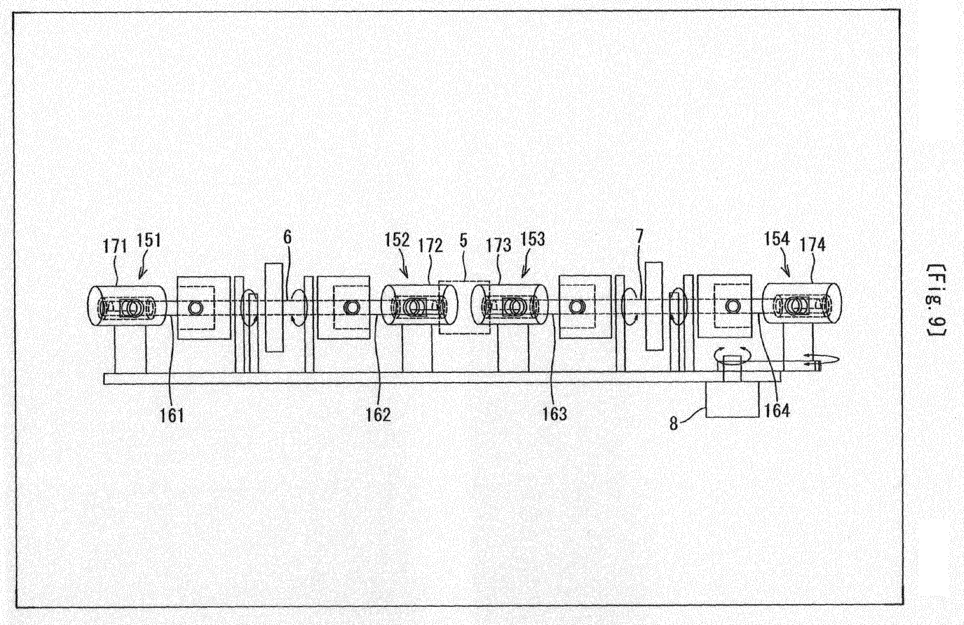

As illustrated in Figs. 7 to 9, the one pair of plunger pumps 151 and 152 have a mirror-symmetrical structure with respect to the plane (plane orthogonal to the sheet of Figs. 7 to 9) orthogonal to the rotation shaft 50 of the synchronization motor 5. In detail, as illustrated in Figs. 7 and 8, the plunger pumps 151 and 152 have a mirror-symmetrical structure with respect to a plane 101 orthogonal to the rotation shaft 50 of the synchronization motor 5 and including the middle in a direction along the axial direction of the drive joint 6. Therefore, the angles at which the plungers 161 and 162 incline with respect to the drive joint 6, the positions of the ports of the cylinders 171 and 172, and the like are in a mirror-symmetrical state.

Thus, the phases of the plunger pumps 151 and 152 are different from each other by 180° with respect to the rotation of the drive joint 6. More specifically, the plunger pump 152 is sucking a fresh dialysate while the plunger pump 151 is delivering a fresh dialysate and the plunger pump 152 is delivering a fresh dialysate while the plunger pump 151 is sucking a fresh dialysate. Thus, the pair of plunger pumps 151 and 152 alternately and continuously perform the delivery of a fresh dialysate by the plunger pump 151 and the delivery of a fresh dialysate by the plunger pump 152.

In the used dialysate discharge line 42, a part of the line between the blood purifier 1 and a waste tank (not illustrated) has a parallel structure in which the line is divided into two lines. In the used dialysate discharge line 42 forming the parallel structure, plunger pumps 153 and 154 are individually disposed in the parallel lines so as to form one pair.

To the pair of plunger pumps 153 and 154, rotation is transmitted through a drive joint 7 (an example of the second drive joint) from the rotation shaft 50 of the synchronization motor 5. The drive joint 7 is connected to one end side of each of plungers 163 and 164 of the plunger pumps 153 and 154, respectively. The plungers 163 and 164 reciprocate in cylinders 173 and 174, respectively, by drive transmitted from the drive joint 7. Axial directions 192 of the plungers 163 and 164 incline (cross) with respect to an axial direction 194 of the drive joint 7. The strokes of the plungers 163 and 164 which reciprocate by drive transmitted from the drive joint 7 are determined depending on the inclination angle θ between the axial directions 192 of the plungers 163 and 164 and the axial direction 194 of the drive joint 7. More specifically, when the inclination angle θ is large, the strokes of the plungers 163 and 164 become large and, when the inclination angle θ is small, the strokes of the plungers 161 and 162 become small.

The inclination angle θ with respect to the drive joint 6 or the drive joint 7 in each of the plungers 161, 162, and 163 of each of the plunger pumps 151, 152, and 153 is fixed but the inclination angle θ with respect to the drive joint 7 of the plunger 164 of the plunger pump 154 is variable. The inclination angle θ of the plunger 164 is adjusted by the drive of the angle adjustment motor 8.

In detail, the drive joint 7 is provided with a bearing socket. To the bearing socket, a bearing is attached. The bearings each are provided with a through-hole in the central portion. One end of operation shafts extending from the plungers 163 and 164 of the plunger pumps 153 and 154, respectively, is slidably inserted into and passed through the through-holes. The operation shafts are fixed so that that the other ends thereof are perpendicular to the surface of the plungers 163 and 164. The rotation of the rotation shaft 50 of the synchronization motor 5 is transmitted to the plungers 163 and 164 through the drive joint 7 and the operation shafts and the strokes d1 and d2 are individually generated in the plunger pump 153 and 154 according to the inclination angle θ. Thus, the plungers 163 and 164 reciprocate in the strokes d1 and d2 while rotating in the cylinders 173 and 174, respectively. The same applies to the transmission of the drive to the plunger pumps 151 and 152 from the drive joint 6.

A pair of ports communicating with the internal space are provided in each of the cylinders 173 and 174. The pair of ports are disposed at positions different by 180° with respect to the axial directions of the cylinders 173 and 174, respectively, i.e., axial symmetry. Although not illustrated in detail in each figure, the plungers 163 and 164 have a columnar shape sealing the cylinders 173 and 174, respectively, in a fluid-tight manner and the half including the axis line of the columnar shape on the tip side (other end side which is not connected to the drive joint 7) is notched. Due to the fact that the notched portions rotate in the cylinders 173 and 174, one of the pair of ports of the cylinders 173 and 174 is sealed by the plungers 163 and 164, respectively, and the other port is opened by the notched portion.

As illustrated in Figs. 7 to 9, the one pair of plunger pumps 153 and 154 have a mirror-symmetrical structure with respect to the plane (plane orthogonal to the sheet of Figs. 7 to 9) orthogonal to the rotation shaft 50 of the synchronization motor 5. In detail, as illustrated in Figs. 7 and 8, the plunger pumps 153 and 154 have a mirror-symmetrical structure with respect to a plane 102 orthogonal to the rotation shaft 50 of the synchronization motor 5 and including the middle in a direction along the axial direction of the drive joint 7. Therefore, the angles at which the plungers 163 and 164 incline with respect to the drive joint 7, the positions of the ports of the cylinders 173 and 174, and the like are in a mirror-symmetrical state.

Thus, the phases of the plunger pumps 153 and 154 are different from each other by 180° with respect to the rotation of the drive joint 7. More specifically, the plunger pump 154 is delivering a used dialysate while the plunger pump 153 is sucking a used dialysate and the plunger pump 154 is sucking a used dialysate while the plunger pump 153 is delivering a used dialysate. Thus, the pair of plunger pumps 153 and 154 alternately and continuously perform the suction of a used dialysate by the plunger pump 153 and the suction of a used dialysate by the plunger pump 154.

As illustrated in Figs. 7 to 9, the pair of plunger pumps 151 and 152 and the pair of plunger pumps 153 and 154 have a mirror-symmetrical structure with respect to the plane (plane orthogonal to the sheet of Figs. 7 to 9) orthogonal to the rotation shaft 50 of the synchronization motor 5. In detail, as illustrated in Figs. 7 and 8, the pair of plunger pumps 151 and 152 and the pair of plunger pumps 153 and 154 have a mirror-symmetrical structure with respect to a plane 103 including the center of the rotation shaft 50 of the synchronization motor 5. Therefore, the supply of a fresh dialysate to the blood purifier 1 by the plunger pumps 151 and 152 and the discharge of a used dialysate from the blood purifier 1 by the plunger pump 153 and 154 are simultaneously performed in a synchronized manner. Thus, the pulsation of a dialysate in the supply of a fresh dialysate to the blood purifier 1 by the plunger pumps 151 and 152 and the pulsation of a dialysate in the discharge of a used dialysate from the blood purifier 1 by the plunger pumps 153 and 154 are synchronized at the same phase.

The capacities of the cylinders 171, 172, 173, and 174 and the structures of the plungers 161, 162, 163, and 164 of the plunger pumps 151, 152, 153 and 154 are the same. Therefore, when the inclination angle θ of each of the plungers 161, 162, 163, and 164 is the same, the delivery amount of a fresh dialysate or the suction amount of a used dialysate by each of the plunger pumps 151, 152, 153, and 154 is the same. However, due to the fact that the stroke d2 of the plunger 164 of the plunger pump 154 is made variable, the suction amount of a used dialysate per the stroke d2 by the plunger pump 154 can be varied so as to be different from the delivery amount of a fresh dialysate or the suction amount of a used dialysate by the other plunger pumps 151, 152, and 153. Therefore, when the stroke d2 of the plunger pump 154 is made larger than the stroke d1 of the plungers 161 and 162 on the fresh dialysate delivery side, removal of water from blood can be performed in the blood purifier 1 and, when the stroke d2 is made smaller than the stroke d1, backfiltration of blood can be performed in the blood purifier 1.

### [Operational effects of second embodiment]

According to the second embodiment, the delivery of a fresh dialysate from the plunger pump 151 and the delivery of a fresh dialysate from the plunger pump 152 are alternately and continuously performed in the pair of plunger pumps 151 and 152 provided in the parallel fresh dialysate supply lines 41, the suction of a used dialysate into the plunger pump 153 and the suction of a used dialysate into the plunger pump 154 are alternately and continuously performed in the pair of plunger pumps 153 and 154 of the parallel used dialysate discharge lines 42, and further the plunger pumps are synchronized so that the delivery of a fresh dialysate and the suction of s used dialysate simultaneously occur. Therefore, the supply of a fresh dialysate to the blood purifier 1 and the discharge of a used dialysate from the blood purifier 1 are simultaneously performed. Thus, a fresh dialysate without pulsation is supplied to the blood purifier 1.

Moreover, the stroke d2 of the plunger pump 154 of the pair of plunger pumps 153 and 154 of the parallel used dialysate discharge lines 42 is made variable. Therefore, when the stroke d2 of the plunger pump 154 provided in the used dialysate discharge line 42 is adjusted to be smaller than the strokes d1 of the plunger pumps 151 and 152 provided in the fresh dialysate supply line 41, backfiltration of blood can be performed in the blood purifier 1. When the stroke d2 of the plunger pump 154 provided in the used dialysate discharge line is adjusted to be larger than the strokes d1 of the plunger pumps 151 and 152 provided in the fresh dialysate supply line 41, removal of water from blood can be performed in the blood purifier 1.

Moreover, the pair of plunger pumps 151 and 152 are disposed in a mirror-symmetrical manner with respect to the plane 101 orthogonal to the rotation shaft 50 of the synchronization motor 5 and are individually connected to the rotation shaft 50 of the synchronization motor 5 through the drive joint 6. Therefore, the pair of plunger pumps 151 and 152 have a relationship in which the phases are 180° shifted from each other. Therefore, even when the phases of the pair of plunger pumps 151 and 152 are not adjusted, the supply of a fresh dialysate to the blood purifier 1 can be alternately and continuously performed.

Moreover, the pair of plunger pumps 153 and 154 are disposed in a mirror-symmetrical manner with respect to the plane 102 orthogonal to the rotation shaft 50 of the synchronization motor 5 and are individually connected to the rotation shaft 50 of the synchronization motor 5 through the drive joint 7. Therefore, the pair of plunger pumps 153 and 154 have a relationship in which the phases are 180° shifted from each other. Therefore, even when the phases of the pair of plunger pumps 153 and 154 are not adjusted, the discharge of a used dialysate from the blood purifier 1 can be alternately and continuously performed.

The pair of plunger pumps 151 and 152 provided in the fresh dialysate supply line 41 and the pair of plunger pumps 153 and 154 provided in the used dialysate discharge line 42 are disposed in a mirror-symmetrical manner with respect to the plane 103 orthogonal to the rotation shaft 50 of the synchronization motor 5. Therefore, the pair of plunger pumps 151 and 152 and the pair plunger pump 153 and 154 have a relationship in which the phases are 180° shifted from each other. Thus, even when the phases of the pair of plunger pumps 151 and 152 and the phases of the pair plunger pump 153 and 154 are not adjusted, the supply of a fresh dialysate to the blood purifier 1 and the discharge of a used dialysate from the blood purifier can be simultaneously performed.

Moreover, the stroke d2 of the plunger pump 154 provided in the used dialysate discharge line 42 can be adjusted by adjusting the inclination angle θ. Therefore, when the stroke d2 of the plunger pump 154 is made larger than the stroke d1 of the plunger 161 and 162 on the fresh dialysate delivery side, removal of water from blood can be performed in the blood purifier 1 and, when the stroke d2 is made smaller than the stroke d1, backfiltration of blood can be performed in the blood purifier 1.

### [Modification of second embodiment]

In the second embodiment described above, the pair of plunger pumps 151 and 152 provided in the fresh dialysate supply line 41 and the pair of plunger pumps 153 and 154 provided in the used dialysate discharge line 42 are disposed in a mirror-symmetrical manner with respect to the plane 103 orthogonal to the rotation shaft 50 of the synchronization motor 5. However, even when the mirror-symmetrical manner is not always employed, the pair of plunger pumps 151 and 152 and the pair plunger pump 153 and 154 can establish a relationship in which the phases are 180° shifted from each other.

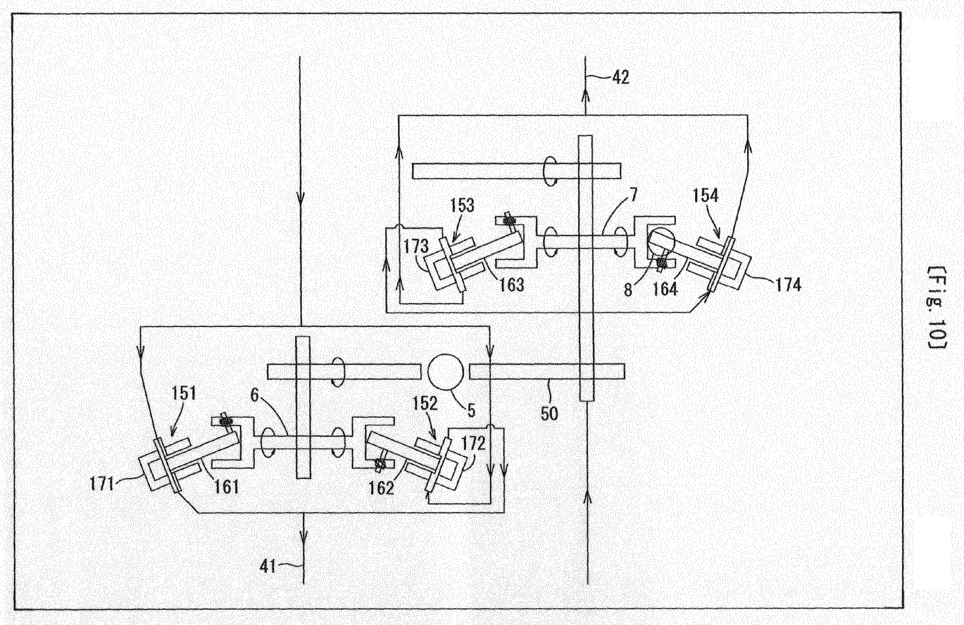

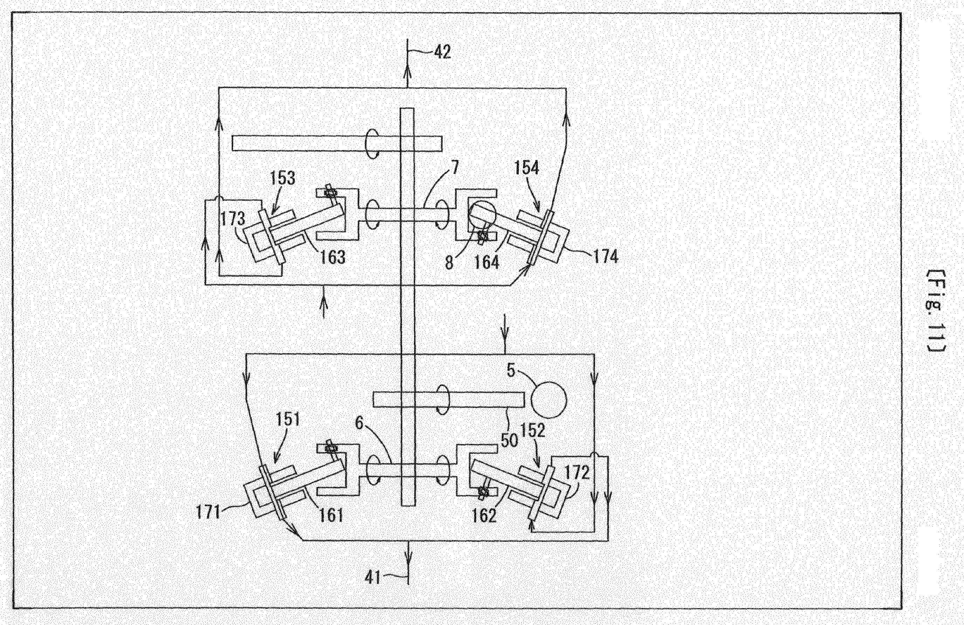

For example, as illustrated in Figs. 10 and 11, the drive joint 6 and the drive joint 7 are not disposed on one rotation axis line and are disposed to be offset to positions different from each other with respect to the rotation shaft 50 of the synchronization motor 5. However, insofar as the drive joint 6 and the drive joint 7 are disposed on one rotation axis line, the supply of a fresh dialysate to the blood purifier 1 and the discharge of a used dialysate from the blood purifier can be simultaneously performed even in the case of the mirror-symmetrical arrangement with respect to the plane 103 and even when the phases of the pair of plunger pumps 151 and 152 and the pair of plunger pumps 153 and 154 are not adjusted. Moreover, the pair of plunger pumps 151 and 152 and the pair of plunger pumps 153 and 154 can be disposed in parallel to each other instead of being disposed in series along the rotation shaft 50 of the synchronization motor 5, a reduction in size of the blood purification device 11 can be achieved. In particular, due to the fact that the pair of plunger pumps 151 and 152 and the pair of plunger pumps 153 and 154 are disposed in a perfect parallel manner as illustrated in Fig. 11, the contribution to a reduction in size of the blood purification device 11 becomes remarkable.

### Reference Signs List

- 1: Blood purifier
- 2: Blood circuit
- 3: Blood pump
- 4: Dialysate line
- 5: Synchronization motor
- 6, 7: Drive joint
- 8: Angle adjustment motor
- 10, 11: Blood purification device
- 41: Fresh dialysate supply line
- 42: Used dialysate discharge Line
- 50: Rotation shaft
- 51, 52, 151, 152, 153, 154: Plunger pump
- 61, 62, 161, 162, 163, 164: Plunger
- 71, 72, 171, 172, 173, 174: Cylinder

## Claims

1. A blood purification device comprising:
a blood purifier (1);
a blood circuit (2) connected to the blood purifier;
a blood pump (3) for generating blood flow in the blood circuit;
a dialysate line (4) having a fresh dialysate supply line (41) and a used dialysate discharge line (42) individually connected to the blood purifier; and
plunger pumps (51, 52) individually provided in the fresh dialysate supply line (41) and the used dialysate discharge line (42) so as to form one pair, wherein
the plunger pump (51) provided in the fresh dialysate supply line (41) and the plunger pump (52) provided in the used dialysate discharge line (42) are synchronized so that delivery of a fresh dialysate by the plunger pump (51) provided in the fresh dialysate supply line (41) and suction of a used dialysate by the plunger pump (52) provided in the used dialysate discharge line (42) are simultaneously performed,
wherein a stroke of one of the plunger pumps of the pair of plunger pumps (51, 52) individually provided in the fresh dialysate supply line (41) and the used dialysate discharge line (42) is variable and can be made larger and smaller than the stroke of the other plunger pump.

2. The blood purification device according to Claim 1 further comprising:
a synchronization motor (5), and
drive joints (6, 7) which rotate by driving force given from the synchronization motor (5), wherein
the pair of plunger pumps (51, 52) are disposed in a mirror-symmetrical manner with respect to a plane orthogonal to a rotation shaft of the synchronization motor (5) and are connected to the synchronization motor by the drive joints (6, 7) .

3. The blood purification device according to Claim 1, wherein in the plunger pump in which the stroke is variable of the pair of plunger pumps, an inclination angle of a shaft of a plunger with respect to the rotation shaft of the synchronization motor (5) is adjustable.

4. The blood purification device according to Claim 3 further comprising:
an angle adjustment motor (8) varying the inclination angle of the shaft of the plunger.

5. The blood purification device according to Claim 1, 3, or 4, wherein a stroke of the plunger pump (52) provided in the used dialysate discharge line (42) is variable.

6. The blood purification device according to Claim 5, wherein a rotation radius of the drive joint (6, 7) on a used dialysate suction side is larger than a rotation radius of the drive joint on a fresh dialysate delivery side.

7. The blood purification device according to any one of Claims 1 to 6, wherein the plunger pump is a valveless plunger pump.

8. The blood purification device according to any one of Claims 1 to 7, wherein another pair of plunger pumps having a 180° shifted phase are disposed in parallel to the dialysate line.

9. A blood purification device comprising:
a blood purifier (1);
a blood circuit (2) connected to the blood purifier;
a blood pump (3) for generating blood flow in the blood circuit;
a dialysate line (4) having a fresh dialysate supply line (41) and a used dialysate discharge line (42) which are individually connected to the blood purifier (1) so as to be at least partially in parallel to each other; and
a pair of plunger pumps (151-154) provided in each of the parallel fresh dialysate supply lines (41) and the parallel used dialysate discharge lines (42), wherein
the pair of plunger pumps (151, 152) individually provided in the parallel fresh dialysate supply lines alternately and continuously perform delivery of a fresh dialysate by one plunger pump of the pair of plunger pumps and delivery of a fresh dialysate by the other plunger pump,
the pair of plunger pumps (153, 154) provided in the parallel used dialysate discharge lines alternately and continuously perform suction of a used dialysate by one plunger pump of the pair of plunger pumps and suction of a used dialysate by the other plunger pump, and
the pair of plunger pumps (151, 152) provided in the fresh dialysate supply line and the pair of plunger pumps (153, 154) provided in the used dialysate discharge line are synchronized so that delivery of a fresh dialysate by the pair of plunger pumps (151, 152) provided in the fresh dialysate supply line and suction of a used dialysate by the pair of plunger pumps (153, 154) provided in the used dialysate discharge line are simultaneously performed, wherein a stroke of one of the plunger pumps of the pair of plunger pumps (154) provided in the parallel used dialysate discharge lines is variable and can be made larger and smaller than the strokes of the pair of plunger pumps (151, 152) provided in the fresh dialysate supply line.

10. The blood purification device according to Claim 9 further comprising:
a synchronization motor (5); and
a first drive joint which rotates by driving force given from the synchronization motor, wherein
the pair of plunger pumps (151, 152) provided in the parallel fresh dialysate supply lines are disposed in a mirror-symmetrical manner with respect to a plane orthogonal to a rotation shaft of the synchronization motor and are connected to the synchronization motor by the first drive joint.

11. The blood purification device according to any one of Claims 9 or 10 further comprising:
a synchronization motor; and
a second drive joint which rotates by driving force given from the synchronization motor, wherein
the pair of plunger pumps (153, 154) provided in the parallel used dialysate discharge lines are disposed in a mirror-symmetrical manner with respect to a plane orthogonal to the rotation shaft of the synchronization motor and are connected to the synchronization motor by the second drive joint.

12. The blood purification device according to Claim 11, wherein the pair of plunger pumps (151, 152) provided in the parallel fresh dialysate supply lines and the pair of plunger pumps (153, 154) provided in the parallel used dialysate discharge lines are disposed in a mirror-symmetrical manner with respect to a plane orthogonal to the rotation shaft of the synchronization motor.

13. The blood purification device according to Claim 9 further comprising:
a synchronization motor; and
a second drive joint which rotates by driving force given from the synchronization motor, wherein
the pair of plunger pumps (153, 154) provided in the parallel used dialysate discharge lines are connected to the synchronization motor by the second drive joint, and
in the plunger pump (154) in which the stroke is variable of the pair of plunger pumps individually provided in the parallel used dialysate discharge lines, an inclination angle of a shaft of a plunger with respect to the rotation shaft of the synchronization motor is adjustable.

14. The blood purification device according to Claim 13 further comprising:
an angle adjustment motor varying the inclination angle of the shaft of the plunger.

## Patentansprüche

1. Blutreinigungsvorrichtung umfassend:
einen Blutreiniger (1);
einen Blutkreislauf (2), welcher mit dem Blutreiniger verbunden ist;
eine Blutpumpe (3), um einen Blutfluss in dem Blutkreislauf zu erzeugen;
eine Dialysat-Leitung (4), welche eine Zuführungsleitung (41) für frisches Dialysat und eine Abflussleitung (42) für verbrauchtes Dialysat aufweist, welche jeweils mit dem Blutreiniger verbunden sind; und
Kolbenpumpen (51, 52), welche jeweils in der Zuführungsleitung (41) für frisches Dialysat und in der Abflussleitung (42) für verbrauchtes Dialysat vorhanden sind, um so ein Paar auszubilden,
wobei die Kolbenpumpe (51), welche in der Zuführungsleitung für frisches Dialysat vorhanden ist, und die Kolbenpumpe (52), welche in der Abflussleitung (42) für verbrauchtes Dialysat vorhanden ist, synchronisiert sind, so dass eine Abgabe von frischem Dialysat durch die Kolbenpumpe (51), welche in der Zuführungsleitung (41) für frisches Dialysat vorhanden ist, und ein Ansaugen von verbrauchtem Dialysat durch die Kolbenpumpe (52), welche in der Abflussleitung (42) für verbrauchtes Dialysat vorhanden ist, gleichzeitig ausgeführt werden,
wobei ein Hub von einer der Kolbenpumpen des Paares von Kolbenpumpen (51, 52), welche jeweils in der Zuführungsleitung (41) für frisches Dialysat und in der Abflussleitung (42) für verbrauchtes Dialysat vorhanden sind, variabel ist und größer und kleiner als der Hub der anderen Kolbenpumpe ausgebildet sein kann.

2. Blutreinigungsvorrichtung nach Anspruch 1, darüber hinaus umfassend:
einen Synchronisationsmotor (5), und
Antriebsverbindungen (6, 7), welche sich durch eine Antriebskraft von dem Synchronisationsmotor (5) drehen,
wobei das Paar von Kolbenpumpen (51, 52) in einer spiegelsymmetrischen Weise bezüglich einer Ebene orthogonal zu einer Drehwelle des Synchronisationsmotors (5) angeordnet und durch die Antriebsverbindungen (6, 7) mit dem Synchronisationsmotor verbunden sind.

3. Blutreinigungsvorrichtung nach Anspruch 1, wobei bei der Kolbenpumpe, bei welcher der Hub variabel bezüglich des Paares von Kolbenpumpen ist, ein Neigungswinkel einer Welle eines Kolbens bezüglich der Drehwelle des Synchronisationsmotors (5) einstellbar ist.

4. Blutreinigungsvorrichtung nach Anspruch 3, darüber hinaus umfassend:
einen Winkeleinstellungsmotor (8), welcher den Neigungswinkel der Welle des Kolbens verändert.

5. Blutreinigungsvorrichtung nach Anspruch 1, 3 oder 4, wobei ein Hub der Kolbenpumpe (52), welche in der Abflussleitung (42) für verbrauchtes Dialysat vorhanden ist, variabel ist.

6. Blutreinigungsvorrichtung nach Anspruch 5, wobei ein Rotationsradius der Antriebsverbindung (6, 7) auf einer Ansaugseite für verbrauchtes Dialysat größer als eine Rotationsradius der Antriebsverbindung auf einer Abgabeseite für frisches Dialysat ist.

7. Blutreinigungsvorrichtung nach einem der Ansprüche 1 bis 6, wobei die Kolbenpumpe eine ventillose Kolbenpumpe ist.

8. Blutreinigungsvorrichtung nach einem der Ansprüche 1 bis 7, wobei ein anderes Paar von Kolbenpumpen, welches eine um 180° verschobene Phase aufweist, parallel zu der Dialysat-Leitung angeordnet ist.

9. Blutreinigungsvorrichtung umfassend:
einen Blutreiniger (1);
einen Blutkreislauf (2), welcher mit dem Blutreiniger verbunden ist;
eine Blutpumpe (3), um einen Fluss in dem Blutkreislauf zu erzeugen;
eine Dialysat-Leitung (4), welche eine Zuführungsleitung (41) für frisches Dialysat und eine Abflussleitung (42) für verbrauchtes Dialysat aufweist, welche jeweils mit dem Blutreiniger (1) verbunden sind, so dass sie zumindest teilweise parallel zueinander sind; und
ein Paar von Kolbenpumpen (151-154), welche in jeder der parallelen Zuführungsleitungen (41) für frisches Dialysat und den parallelen Abflussleitungen (42) für verbrauchtes Dialysat vorhanden sind,
wobei das Paar von Kolbenpumpen (151, 152), welche jeweils in den parallelen Zuführungsleitungen für frisches Dialysat vorhanden sind, abwechselnd und kontinuierlich eine Abgabe von frischem Dialysat durch eine Kolbenpumpe des Paares von Kolbenpumpen und eine Abgabe von frischem Dialysat durch die andere Kolbenpumpe bereitstellen,
wobei das Paar von Kolbenpumpen (153, 154), welche parallel in den Abflussleitungen für verbrauchtes Dialysat vorhanden sind, abwechselnd und kontinuierlich ein Ansaugen von verbrauchtem Dialysat durch eine Kolbenpumpe des Paares von Kolbenpumpen und ein Ansaugen von verbrauchtem Dialysat durch die andere Kolbenpumpe ausführen, und
wobei das Paar von Kolbenpumpen (151, 152), welche in der Zuführungsleitung für frisches Dialysat vorhanden sind, und das Paar von Kolbenpumpen (153, 154), welche in der Abflussleitung für verbrauchtes Dialysat vorhanden sind, synchronisiert sind, so dass eine Abgabe von frischem Dialysat durch das Paar von Kolbenpumpen (151, 152), welche in der Zuführungsleitung für frisches Dialysat vorhanden sind, und ein Ansaugen von verbrauchtem Dialysat durch das Paar von Kolbenpumpen (153, 154), welche in der Abflussleitung für verbrauchtes Dialysat vorhanden sind, gleichzeitig ausgeführt werden, wobei ein Hub von einer der Kolbenpumpen des Paares von Kolbenpumpen (154), welche in den parallelen Abflussleitungen für verbrauchtes Dialysat vorhanden sind, variabel ist und größer und kleiner als die Hübe des Paares von Kolbenpumpen (151, 152), welche in der Zuführungsleitung für frisches Dialysat vorhanden sind, ist.

10. Blutreinigungsvorrichtung nach Anspruch 9, darüber hinaus umfassend:
einen Synchronisationsmotor (5); und
eine erste Antriebsverbindung, welche sich durch eine Antriebskraft von dem Synchronisationsmotor dreht,
wobei das Paar von Kolbenpumpen (151, 152), welche in den parallelen Zuführungsleitungen für frisches Dialysat vorhanden sind, in einer spiegelsymmetrischen Weise bezüglich einer Ebene orthogonal zu einer Drehwelle des Synchronisationsmotors angeordnet und durch die erste Antriebsverbindung mit dem Synchronisationsmotor verbunden sind.

11. Blutreinigungsvorrichtung nach einem der Ansprüche 9 oder 10, darüber hinaus umfassend:
einen Synchronisationsmotor; und
eine zweite Antriebsverbindung, welche sich durch eine Antriebskraft von dem Synchronisationsmotor dreht,
wobei das Paar von Kolbenpumpen (153, 154), welche in den parallelen Abflussleitungen für verbrauchtes Dialysat vorhanden sind, in einer spiegelsymmetrischen Weise bezüglich einer Ebene orthogonal zu der Drehwelle des Synchronisationsmotors angeordnet und mit dem Synchronisationsmotor durch die zweite Antriebsverbindung verbunden sind.

12. Blutreinigungsvorrichtung nach Anspruch 11, wobei das Paar von Kolbenpumpen (151, 152), welche in den parallelen Zuführungsleitungen für frisches Dialysat vorhanden sind, und das Paar von Kolbenpumpen (153, 154), welche in den parallelen Abflussleitungen für verbrauchtes Dialysat vorhanden sind, in einer spiegelsymmetrischen Weise bezüglich einer Ebene orthogonal zu der Drehwelle des Synchronisationsmotors angeordnet sind.

13. Blutreinigungsvorrichtung nach Anspruch 9, darüber hinaus umfassend:
einen Synchronisationsmotor; und
eine zweite Antriebsverbindung, welche sich durch eine Antriebskraft von dem Synchronisationsmotor dreht,
wobei das Paar von Kolbenpumpen (153, 154), welche in den parallelen Abflussleitungen für verbrauchtes Dialysat vorhanden sind, mit dem Synchronisationsmotor durch die zweite Antriebsverbindung verbunden sind, und
wobei bei der Kolbenpumpe (154), bei welcher der Hub variabel bezüglich des Paares von Kolbenpumpen ist, welche jeweils in den parallelen Abflussleitungen für verbrauchtes Dialysat vorhanden sind, ein Neigungswinkel einer Welle eines Kolbens bezüglich der Drehwelle des Synchronisationsmotors einstellbar ist.

14. Blutreinigungsvorrichtung nach Anspruch 13, darüber hinaus umfassend:
einen Winkeleinstellungsmotor, welcher den Neigungswinkel der Welle des Kolbens verändert.

## Revendications

1. Dispositif de purification du sang, comprenant :
un purificateur du sang (1) ;
un circuit sanguin (2) raccordé au purificateur du sang ;
une pompe sanguine (3) pour générer un flux de sang dans le circuit sanguin ;
une conduite de dialysat (4) ayant une conduite d'alimentation en dialysat frais (41) et une conduite de décharge de dialysat usagé (42) raccordées individuellement au purificateur de sang ; et
des pompes à piston (51, 52) disposées individuellement dans la conduite d'alimentation en dialysat frais (41) et dans la conduite de décharge de dialysat usagé (42) de manière à former une paire, dans lequel :
la pompe à piston (51) disposée dans la conduite d'alimentation en dialysat frais (41) et la pompe à piston (52) disposée dans la conduite de décharge de dialysat usagé (42) sont synchronisées de sorte que la fourniture d'un dialysat frais par la pompe à piston (51) disposée dans la conduite d'alimentation en dialysat frais (41) et l'aspiration d'un dialysat usagé par la pompe à piston (52) disposée dans la conduite de décharge de dialysat usagé (42) soient effectuées simultanément,
dans lequel une course de l'une des pompes à piston de la paire de pompes à piston (51, 52) individuellement disposées dans la conduite d'alimentation en dialysat frais (41) et dans la conduite de décharge de dialysat usagé (42) est variable et peut être allongée et réduite par rapport à la course de l'autre pompe à piston.

2. Dispositif de purification du sang selon la revendication 1, comprenant en outre :
un moteur de synchronisation (5), et
des joints d'entraînement (6, 7) qui tournent sous l'action d'une force d'entraînement appliquée par le moteur de synchronisation (5), dans lequel
la paire de pompes à piston (51, 52) est disposée en mode de symétrie spéculaire par rapport à un plan orthogonal à un arbre de rotation du moteur de synchronisation (5) et est raccordée au moteur de synchronisation par les joints d'entraînement (6, 7).

3. Dispositif de purification du sang selon la revendication 1, dans lequel, dans la pompe à piston dans laquelle la course est variable de la paire de pompes à piston, un angle d'inclinaison d'un arbre d'un piston par rapport à l'arbre de rotation du moteur de synchronisation (5) est ajustable.

4. Dispositif de purification du sang selon la revendication 3, comprenant en outre :
un moteur d'ajustement d'angle (8) faisant varier l'angle d'inclinaison de l'arbre du piston.

5. Dispositif de purification du sang selon la revendication 1, 3 ou 4, dans lequel une course de la pompe à piston (52) disposée dans la conduite de décharge de dialysat usagé (42) est variable.

6. Dispositif de purification du sang selon la revendication 5, dans lequel un rayon de rotation du joint d'entraînement (6, 7) sur un côté d'aspiration de dialysat usagé est plus grand qu'un rayon de rotation du joint d'entraînement sur un côté d'alimentation en dialysat frais.

7. Dispositif de purification du sang selon l'une quelconque des revendications 1 à 6, dans lequel la pompe à piston est une pompe à piston sans vanne.

8. Dispositif de purification du sang selon l'une quelconque des revendications 1 à 7, dans lequel une autre paire de pompes à piston ayant une phase décalée de 180° est disposée en parallèle avec la conduite de dialysat.

9. Dispositif de purification du sang, comprenant :
un purificateur du sang (1) ;
un circuit sanguin (2) raccordé au purificateur du sang ;
une pompe sanguine (3) pour générer un flux sanguin dans le circuit sanguin ;
une conduite de dialysat (4) ayant une conduite d'alimentation en dialysat frais (41) et une conduite de décharge de dialysat usagé (42) qui sont raccordées individuellement au purificateur de sang (1) de manière à être au moins en partie parallèles l'une à l'autre ; et
une paire de pompes à piston (151, 154) disposées dans chacune des conduites d'alimentation en dialysat frais parallèles (41) et des conduites de décharge de dialysat usagé parallèles (42), dans lequel :
la paire de pompes à piston (151, 152) est individuellement disposée dans les conduites d'alimentation en dialysat frais parallèles en alternance et effectue en continu la fourniture d'un dialysat frais par une pompe à piston de la paire de pompes à piston et la fourniture d'un dialysat frais par l'autre pompe à piston,
la paire de pompes à piston (153, 154) disposée dans les conduites de décharge de dialysat usagé parallèles effectue en alternance et en continu l'aspiration d'un dialysat usagé par une pompe à piston de la paire de pompes à piston et l'aspiration d'un dialysat usagé par l'autre pompe à piston, et
la paire de pompes à piston (151, 152) disposée dans la conduite d'alimentation en dialysat frais et la paire de pompes à piston (153, 154) disposée dans la conduite de décharge de dialysat usagé sont synchronisées de sorte que la fourniture d'un dialysat frais par la paire de pompes à piston (151, 152) disposées dans la conduite d'alimentation en dialysat frais et l'aspiration d'un dialysat usagé par la paire de pompes à piston (153, 154) disposées dans la conduite de décharge de dialysat usagé soient effectuées simultanément, dans lequel une course de l'une des pompes à piston de la paire de pompes à piston (154) disposées dans les conduites de décharge de dialysat usagé parallèles est variable et peut être allongée et réduite par rapport aux courses de la paire de pompes à piston (151, 152) disposées dans la conduite d'alimentation en dialysat frais.

10. Dispositif de purification du sang selon la revendication 9, comprenant en outre :
un moteur de synchronisation (5) ; et
un premier joint d'entraînement qui tourne sous l'action d'une force d'entraînement appliquée par le moteur de synchronisation, dans lequel
la paire de pompes à piston (151, 152) disposée dans les conduites d'alimentation en dialysat frais parallèles est aménagée en mode de symétrie spéculaire par rapport à un plan orthogonal à un arbre de rotation du moteur de synchronisation et est raccordée au moteur de synchronisation par le premier joint d'entraînement.

11. Dispositif de purification du sang selon l'une quelconque des revendications 9 ou 10, comprenant en outre :
un moteur de synchronisation ; et
un second joint d'entraînement qui tourne sous l'action d'une force d'entraînement appliquée par le moteur de synchronisation, dans lequel
la paire de pompes à piston (153, 154) disposée dans les conduites de décharge de dialysat usagé parallèles est aménagée en mode de symétrie spéculaire par rapport à un plan orthogonal à un arbre de rotation du moteur de synchronisation et est raccordée au moteur de synchronisation par le second joint d'entraînement.

12. Dispositif de purification du sang selon la revendication 11, dans lequel la paire de pompes à piston (151, 152) disposée dans les conduites d'alimentation en dialysat frais parallèles et la paire de pompes à piston (153, 154) disposées dans les conduites de décharge de dialysat usagé parallèles sont aménagées en mode de symétrie spéculaire par rapport à un plan orthogonal à l'arbre de rotation du moteur de synchronisation.

13. Dispositif de purification du sang selon la revendication 9, comprenant en outre :
un moteur de synchronisation ; et
un second joint d'entraînement qui tourne sous l'action d'une force d'entraînement appliquée par le moteur de synchronisation, dans lequel
la paire de pompes à piston (153, 154) disposée dans les conduites de décharge de dialysat usagé parallèles est raccordée au moteur de synchronisation par le second joint d'entraînement, et
dans la pompe à piston (154) dans laquelle la course est variable de la paire de pompes à piston disposées individuellement dans les conduites de décharge de dialysat usagé, un angle d'inclinaison d'un arbre d'un piston par rapport à l'arbre de rotation du moteur de synchronisation est ajustable.

14. Dispositif de purification du sang selon la revendication 13, comprenant en outre :
un moteur d'ajustement d'angle faisant varier l'angle d'inclinaison de l'arbre du piston.
